# EUROPEAN PATENT SPECIFICATION

(11) **EP 0 813 876 B1**
(45) Date of publication and mention of the grant of the patent: **27.03.2002**
(21) Application number: 97109152.5
(22) Date of filing: 05.06.1997
(51) Int. Cl.: A61K 38/13, A61K 47/14, A61K 47/08, A61K 9/48

(54) **Cyclosporin-containing soft capsule preparations**
Cyclosporin enthaltende Weichkapselpräparate
Préparations contenant de cyclosporine pour capsules molles

(30) Priority: 19.06.1996 KR 9622417
(43) Date of publication of application: 29.12.1997
(73) Proprietor: Novartis AG, 4056 Basel (CH)
(72) Inventor: Woo, Jong Soo, Jangan-gu, Suwon-shi, Kyunggi-do (KR)

(56) References cited:
- EP-A- 0 712 631
- US-A- 5 589 455

## Description

### Background of the Invention

### 1. Field of the invention

The present invention relates to a soft capsule preparation containing cyclosporin as an active ingredient. More specifically, the present invention relates to a soft capsule preparation containing cyclosporin as an active ingredient; propylene carbonate or polyethylene glycol or a mixture thereof as a cosurfactant; one or a mixture of two or more selected from the group consisting of an esterified compound of fatty acid and primary alcohol, medium chain fatty acid triglyceride and fatty acid monoglyceride as an oil component; and a surfactant having HLB (Hydrophilic-lipophilic balance) value of 8 to 17.

### 2. Background art

Cyclosporin is a specific macromolecular (molecular weight 1202.64) cyclic peptide compound consisting of 11 amino acids, which has a broad spectrum of useful pharmacological activities, particularly an immuno-suppressive activity and an anti-inflammatory activity. Therefore, cyclosporin has been used for suppression of inherent immunological responses of the living body, which are caused by tissue and organ transplantation, for example, transplantation of heart, lung, liver, kidney, pancreas, bone marrow, skin and cornea. In addition, cyclosporin has proved useful for the suppression of hematological disorders, such as anemia, various autoimmune diseases, such as systemic lupus erythematosus and idiopathic malabsorption syndrome, and inflammatory diseases such as arthritis, rheumatoid disorder, etc. Cyclosporin has also been useed in the treatment of protozoal diseases such as malaria, schistosomiasis, etc., and quite recently in cancer chemotherapy.

Cyclosporin is highly lipophilic and hydrophobic. Therefore, cyclosporin is sparingly soluble in water, whereas well dissolved in organic solvents, such as methanol, ethanol, acetone, ether, chloroform and the like. Due to its limited solubility in water, the bioavailability of orally administered cyclosporin is extremely low and may be highly dependent on the condition of the patient. Accordingly, it is very difficult to retain an effective therapeutic concentration. Moreover, cyclosporin may have considerable side effects such as nephrotoxicity. Cyclosporin can thus be formulated into a preparation for oral administration only with great difficulty. Accordingly, numerous studies have been extensively conducted to find a cyclosporin preparation effective for oral administration, that is, a preparation which provides both uniform dosage and bioavailability of the active component.

In the prior art, preparations of cyclosporin suitable for oral administration of sparingly water-soluble cyclosporin have been usually formulated in the form of an emulsion by combining cyclosporin with a surfactant, an oil and a cosurfactant. For example, U.S. Patent No. 4,388,307, which issued on June 14, 1983, discloses a liquid formulation of cyclosporin that includes ethanol as a cosurfactant, olive oil as a vegetable oil, and a trans-esterification product of a natural vegetable oil triglyceride and a polyalkylene polyol as a surfactant. However, since this liquid formulation is administered as an aqueous dilution, it is both inconvenient to administer and difficult to administer in a uniform dosage.

In order to mitigate the inconvenience of diluting a cyclosporin liquid composition with water prior to oral administration, an emulsion concentrate of the liquid composition has been formulated into a soft capsule preparation, which is now commercially available as Sandimmun® . In this preparation, the cyclosporin soft capsule contains a large amount of ethanol as a cosurfactant in order co solubilize cyclosporin. However, since ethanol permeates the gelatin shell of the capsule co volatilize even at normal temperature, the constitutional ratio of the contents of soft capsules may greatly vary during storage. The reduced ethanol content may in turn result in crystallization of cyclosporin and a significant variation in the bioavailability of cyclosporin. The variation with storage time of the bioavailability of the active component makes it quite difficult to determine the dosage needed to provide a desired therapeutic effect.

In an effort to prevent the volatilization of ethanol from soft capsule preparations during storage and distribution, the soft capsule preparations are wrapped in specialized packaging materials, such as aluminum-aluminum blister packaging. However, great variations in the composition of the capsules and consequent variations in cyclosporin bioavailability may occur despite such special packaging, which itself significantly increases the price of the preparation.

Recently, many researchers have attempted to develope a cyclosporin preparation which has a stability during the storage and further exhibits no substantial change in biological availability and no substantial difference in biological availability between individual subjects, so that the bioloical effect of cyclosporin can be uniformly maintained. One of the preparations developed for this purpose is disclosed in Korean Laid-open Patent Publication No. 93-113. This preparation has been sold under the trademark Sandimmun Neoral® . However, since this preparation also uses ethanol as a cosurfactant, it shares some of the disadvantages of prior ethanol-containing preparations, that is, its storage stability is poor, and its ethanol content changes with time to cause the precipitation of cyclosporin and the lowering of cyclosporin bioavailability.

Accordingly, the present inventors have studied numerous combinations of various surfactants, oil components and cosurfactants to find out a cyclosporin composition which is stable, and provides higher bioavailability and lower difference in blood levels between individual subjects than those of cyclosporin compositions of the prior art. As a result of these investigations, we identified that the cyclosporin composition as defined below satisfies the above-mentioned requirements, and thus completed the present invention.

Therefore, it is an object of the present invention to provide a composition suitable for formulation into soft capsules, which contains cyclosporin as an active ingredient; propylene carbonate or polyethylene glycol or a mixture thereof as a cosurfactant; an oil component as defined below; and a surfactant.

It is a further object of the present invention to provide a soft capsule preparation comprising cyclosporin as an active ingredient; propylene carbonate or polyethylene glycol or a mixture thereof as a cosurfactant; one or a mixture of two or more selected from the group consisting of an esterified compound of fatty acid and primary alcohol, medium chain fatty acid triglyceride and fatty acid monoglyceride as an oil component; and a surfactant having HLB (Hydrophilic-lipophilic balance) value of 8 to 17.

### DISCLOSURE OF INVENTION

In one aspect, the present invention relates to a cyclosporin-concaining soft gelatin capsule which has a high storage stability to exhibit little variation of the composition over time, and exhibits greater bioavailability than prior art preparations, and which contains a composition comprising cyclosporin as an active ingredient; propylene carbonate or polyethylene glycol or a mixture thereof as a cosurfactant; an oil component as defined below; and a surfactant.

More specifically, the present invention relates to a cyclosporin soft capsule preparation, which comprises a composition containing:
(1) cyclosporin as an active ingredient;
(2) propylene carbonate or polyethylene glycol or a mixture thereof as a cosurfactant;
(3) one or a mixture of two or more selected from the group consisting of an esterified compound of fatty acid and primary alcohol, medium chain fatty acid triglyceride and fatty acid monoglyceride as an oil compcnent; and
(4) a surfactant having HLB (Hydrophilic-lipophilic balance) value of 8 to 17.

Cyclosporin, which is used as the pharmaceutically active ingredient in the composition according to the present invention, is a cyclic peptide compound having useful immuno-suppressive activity and anti-inflammatory activity as described above. Although cyclosporin A, B, C, D, G and the like can all be used as the cyclosporin component in the present invention, cyclosporin A is preferred, since its clinical effectiveness and pharmacological properties are well established in the art.

As the cosurfactant which is the second essential component in the composition according to the present invention, propylene carbonate or polyethylene glycol or their mixture can be used.

Propylene carbonate which is used as an essential cosurfactant in the composition of the present invention is a non-volatile and non-hydrophilic component having boiling point of about 242°C. Due to high boiling point of propylene carbonate, it does not volatilize during storage at room temperature or even under high temperature condition for preparation of soft capsules to ensure the stability of the soft capsule preparation. Further, since propylene carbonate is a non-alcoholic substance which does not contain any hydroxy group, it shows low hygroscopic property and shell permeability, and further has a high solubility for cyclosporin.

In addition, in the composition of the present invention polyethylene glycol can also be used as a hydrophilic cosurfactant. Polyethylene glycol is also a non-volatile component having high boiling point of about 330°C. In the composition according to the present invention, although any polyethylene glycol which can be liquified can be used as the hydrophilic cosurfactant component, polyethylene glycol (PEG) having molecular weight of 200 to 600, particularly PEG 200, can be preferably used.

In the present invention, the mixture of propylene carbonate and polyethylene glycol as defined above can also be used as the cosurfactant. when the mixture of polyethylene glycol and propylene carbonate is used as the cosurfactant component in the present invantion, they can be generally combined in the ratio of 1:1-5, preferably 1:1-3, most preferably 1:1-2, on the basis of weight.

In the present invention, the use of propylene carbonate and polyethylene glycol as the cosurfactant provides certain advantages. That is, the stability of the cyclosporin-containing composition during storage is improved and therefore the contents of the components contained therein are substantially uniformly maintained. Furthermore, the use of propylene carbonate as non-hydrophilic cosurfactant component can more increase the solubility of the active ingredient cyclosporin and inhibit the inflow of water from the gelatin capsule shell into the composition to provide a more stable composition.

In the composition of the present invention, the cosurfactant is used preferably in the ratio of 0.1 co 10 parts by weight, more preferably 0.5 to 8 parts by weight, and most preferably 1 to 5 parts by weight, per 1 part by weight of cyclosporin.

The third component used in the composition according to the present invention is an oil component. As the oil component in the present invention, one or a mixture or two or more selected from the group consisting of esterified compounds of fatty acid and primary alcohol, medium chain fatty acid triglycerides and fatty acid monoglycerides can be used. The esterified compound of fatty acid and primary alcohol which can preferably be used in the present invention may include an esterified compound of fatty acid having 8 to 20 carbon atoms and primary alcohol having 2 to 3 carbon atoms, for example, isopropyl myristate, isopropyl palmitate, ethyl linoleate, ethyl oleate, etc., with an esterified compound of linoleic acid and ethanol being particularly preferable. In addition, as the medium chain fatty acid triglyceride a triglyceride of saturated fatty acid having 8 to 10 carbon atoms can preferably be used with caprylic/capric acid triglyceride as a vegetable oil triglyceride of saturated fatty acid being most preferably used. The fatty acid monoglyceride which can also be used as the oil component in the present invention includes a monoglyceride of fatty acid having 18 to 20 carbon atoms, particularly monoglyceride of oleic acid.

In the composition according to the present invention, the oil component is used in the ratio of 1 to 10 parts by weight, preferably 2 to 6 parts by weight, per 1 part by weight of cyclosporin. When the oil mixture is used as the oil component in the composition according to the present invention, the mixing ratio of fatty acid monoglyceride : an esterified compound of fatty acid and primary alcohol : medium chain fatty acid triglyceride is generally in the range of 1 : 0.1-5 : 0.1-10, preferably in the range of 1 : 0.1-3.0 : 0.1-3.0, on the basis of weight.

The fourth essential component used in the composition according to the present invention is a surfactant. The suitable surfactants for use in the present invention include any of pharmaceutically acceptable surfactants having HLB (Hydrophilic-lipophilic balance) value of 8 to 17, which are capable of stably emulsifying the lipophilic portion of the composition comprising the cyclosporin-concaining oil component and the hydrophilic portion comprising the cosurfactant in water to form a stable microemulsion. Examples of the preferred surfactant according to the present invention include polyoxyethylene products of hydrogenated vegetable oils, polyoxyethylene-sorbitan-fatty acid esters, and the like, for example, NIKKOL HCO-50, NIKKOL HCO-40, NIKKOL HCO-60, TWEEN 20, TWEEN 21, TWEEN 40, TWEEN 60, TWEEN 80, TWEEN 81, etc. Particularly, a polyoxyethylene (50) hydrogenated castor oil which has been commercialized under the trade mark NIKKOL HCO-50 (Nikko Chemical Co., Ltd.) and a polyoxyethylene (20) sorbitan monolaurate which has been commercialized under the trade mark TWEEN 20 (ICI Chemicals), having an acid value below 1, a saponification value of about 48-56, a hydroxyl value of about 45-55 and pH value (5%) of 4.5-7.0, can be preferably used. The surfactant can include any one of the above-mentioned surfactants alone or, preferably, in a combination of two or more surfactants selected from the above surfactants. In the composition according to the present invention, the surfactants can be used in the ratio of 1 to 10 parts by weight, preferably in the ratio of 2 to 8 parts by weight, per 1 part by weight of cyclosporin. In addition, when the mixture of two surfactants, i.e. polyoxyethylene(50) hydrogenated castor oil and polyoxyethylene (20) sorbitan monolaurate is used in the composition of the present invention, the constitutional ratio of polyoxyethylene (50) hydrogenated castor oil : polyoxyethylene (20) sorbitan monolaurate is preferably in the range of 1 : 0.1-5, more preferably in the range of 1 : 0.5-4, on the basis of weight.

In the composition according to the present invention, the four essential components are present preferably in the ratio of cyclosporin : cosurfactant : oil component : surfactant = 1 : 0.1-10 : 1-10 : 1-10, and more preferably in the ratio of cyclosporin : cosurfactant : oil component : surfactant = 1 : 0.5-8 : 2-6 : 2-8, by weight. In addition to this composition, the working examples which follow provide additional preferable compositions according to the present invention.

The composition of the present invention may be prepared in the form of soft capsules for oral administration.

When the composition of the present invention is preapred in the form of a soft capsule, the composition may be encapsulated in agelatin shell which contains any conventional plasticizer. As the plasticizer which can be included in the gelatin capsule shell, one or mere selected from the group consisting of glycerine, sorbitol, hexanetriol, propylene carbonate, hexane glycol, sorbitans, tetrahydrofuryl alcohol ether, diethylene glycol monoethyl ether, 1,3-dimethyl-2-imidazolidone, dimethylisosorbide, etc. can be used without any limitation. However, it should be understood that the plasticizer which can he used in the present invention is not restricted to those as mentioned above.

In formulating the composition according to the present invention into the soft capsules, the capsule preparation may also contain, if necessary, pharmaceutically acceptable additives which are conventionally utilized in the preparation of soft capsules. Such additives include, for example, lecithin, viscosity regulators, perfumes (e.g. peppermint oil, etc.), antioxidants (e.g. tocopherol, etc.), preservatives (e.g. parabens, etc.), coloring agents, amino acids, etc.

The soft capsule preparation according to the present invention can be prepared in a conventional machine for producing soft capsules by uniformly mixing the cosurfactant, the oil component and the surfactant, dissolving cyclosporin therein while stirring and gently warming the mixture thereby obtained to a temperature of approximately 60°C, and then encapsulating the resulting concentrate, with or without the above-mentioned pharmaceutically acceptable additives.

The present invention will be more specifically illustrated by the following examples. However, it should be understood that the present invention is not limited by these examples in any manner.

### Example 1

| Component | Concent(mg/Cap.) |
|---|---|
| Cyclosporin | 25 |
| propylene carbonate | 50 |
| polyoxyethylene(50) hydrogenated castor oil | 90 |
| polyoxyethylene(20) sorbitan monolaurate | 80 |
| ethyl linoleate | 40 |
| caprylic/capric acid triglyceride | 5 |
| oleic acid monoglyceride | 35 |
| Total | 325mg |

### Example 2

| Component | Content(mg/Cap.) |
|---|---|
| Cyclosporin | 25 |
| propylene carbonate | 100 |
| polyoxyethylene(50) hydrogenated castor oil | 80 |
| polyoxyethylene(20) sorbitan monolaurate | 80 |
| ethyl linoleate | 30 |
| caprylic/capric acid triglyceride | 10 |
| oleic acid monoglyceride | 50 |
| Total | 375mg |

### Example 3

| Component | Content (mg/Cap.) |
|---|---|
| Cyclosporin | 100 |
| propylene carbonate | 200 |
| polyoxyechylene(50) hydrogenated castor oil | 300 |
| polyoxyechylene(20) sorbitan monclaurate | 280 |
| echyl linoleace | 150 |
| caprylic/capric acid triglyceride | 20 |
| oleic acid monoglyceride | 120 |
| Labrafil | 50 |
| Total | 1220mg |

### Example 4

| Component | Content (mg/Cap.) |
|---|---|
| cyclosporin | 25 |
| propylene carbonate | 50 |
| polyoxyethylene(50) hydrogenated castor oil | 90 |
| polyoxyethylene(20) sorbitan monolaurate | 80 |
| caprylic/capric acid triglyceride | 5 |
| oleic acid monoglyceride | 35 |
| Total | 285mg |

### Example 5

| Component | Content (mg/Cap.) |
|---|---|
| Cyclosporin | 25 |
| propylene carbonate | 100 |
| polyoxyethylene(50) hydrogenated castor oil | 90 |
| polyoxyethylene(20) sorbitan monolaurate | 80 |
| ethyl linoleate | 40 |
| caprylic/capric acid triglyceride | 5 |
| oleic acid monoglyceride | 35 |
| Total | 375mg |

### Example 6

| Component | Content(mg/Cap.) |
|---|---|
| Cyclosporin | 25 |
| polyethylene glycol 200 | 35 |
| propylene carbonate | 45 |
| polyoxyethylene (50) hydrogenated castor oil | 30 |
| polyoxyethylene (20) sorbitan monolaurate | 80 |
| ethyl linoleate | 35 |
| caprylic/capric acid triglyceride | 5 |
| oleic acid monoglyceride | 35 |
| Total | 290mg |

### Example 7

| Component | Content(mg/Cap.) |
|---|---|
| Cyclosporin | 25 |
| polyethylene glycol 200 | 50 |
| propylene carbonate | 100 |
| polyoxyethylene (50) hydrogenated castor oil | 35 |
| polyoxyethylene(20) sorbitan monolaurace | 90 |
| ethyl linoleate | 40 |
| caprylic/capric acid triglyceride | 5 |
| oleic acid monoglyceride | 30 |
| Total | 375mg |

### Example 8

| Component | Content (mg/Cap.) |
|---|---|
| Cyclosporin | 25 |
| polyethylene glycol 200 | 25 |
| propylene carbonate | 45 |
| polyoxyethylene(50) hydrogenated castor oil | 50 |
| polyoxyethylene (20) sorbitan monolaurate | 75 |
| ethyl linoleate | 40 |
| caprylic/capric acid triglyceride | 5 |
| oleic acid monoglyceride | 35 |
| Total | 300mg |

### Example 9

| Component | Content(mg/Cap.) |
|---|---|
| Cyclosporin | 25 |
| polyethylene glycol 200 | 20 |
| propylene carbonate | 80 |
| polyoxyethylene(50) hydrogenated castor oil | 35 |
| polyoxyethylene(20) sorbitan monolaurate | 85 |
| ethyl linoleate | 80 |
| caprylic/capric acid triglyceride | 10 |
| oleic acid monoglyceride | 85 |
| Total | 420mg |

### Example 10

| Component | Content(mg/Cap.) |
|---|---|
| Cyclosporin | 25 |
| polyethylene glycol 200 | 70 |
| polyoxyethylene(50) hydrogenated castor oil | 35 |
| polyoxyethylene(20) sorbitan monolaurate | 85 |
| ethyl linoleate | 40 |
| caprylic/capric acid triglyceride | 5 |
| oleic acid monoglyceride | 35 |
| Total | 295mg |

### Example 11

The bioavailability of the soft capsule preparation prepared from the composition of Example 1 according to a conventional manner as the test preparation was compared with the bioavailability of a commercial product containing ethanol, SANDIMMUN® Capsule, as the control preparation to estimate the influence of the cyclosporin preparation according to the present invention on the bioavailability of cyclosporin and its difference between respective subjects.

In this experiment, both the test preparation and the control preparation were administered in an amount of 300mg as cyclosporin per kg of rabbit.

Rabbits were uniformly fed with the conventional rabbit solid feed composition for 4 days or more under the same condition in wire cages. When the oral preparations were administered, rabbits were fasted for 48 hours in a restraint cage made of steel, during which rabbits were allowed to freely take water. A Levin tube of diameter 5mm was interposed by the depth of 30cm through the esophagus after the surface of the Levin tube had been coated with vaseline in order to reduce friction. Each of the test preparation and the control preparation was emulsified with 50ml of water and then introduced into a syringe which is attached to the Levin tube. The ear veins of a rabbit were dilated using xylene and then blood was taken from each rabbit's ear vein before the test and after 0.5, 1, 1.5, 2, 3, 4, 6, 10 and 24 hours by means of heparin-treated disposable syringes. To 1ml of blood thus obtained were added 0.5ml of aqueous saturated sodium chloride solution and 2ml of ether, and then the mixture was shaken for 5 minutes and centrifuged with 5000rpm for 10 minutes to separate the supernatant (ether layer). 1ml of the supernatant was collected and then developed in an activated silica sep-pak® (Waters). The developed sep-pak was washed with 5ml of n-hexane and eluated with 2ml of methanol. The eluate was evaporated to dryness in nitrogen gas under reduced pressure. The residue was analyzed by means of HPLC (High Performance Liquid Chromatography) [HPLC condition : column µ-Bondapak® C₁₈(Waters), mobile phase CH₃CN : MeOH : H₂O = 55 : 15 : 30, detection 210nm, flow rate 1.0ml/min., column temperature 70°C, sensitivity 0.01 Aufs, injection volume 100µl].

The results obtained from the test preparation and the control preparation are illustrated in the following Table 1.

**Table 1.**

| Bioavailability of the test preparation of the present invention and the commercial product (SANDIMMUN® ) | | | | | |
|---|---|---|---|---|---|
| Parameter | Control Prep. (A) | | Test Prep. (B) | | P (B/A) |
| | M±S.D. (n=6) | CV % (S.D./M) | M±S.D. (n=6) | CV % (S.D./M) | |
| AUC (µg·hr/ml) | 13.5±10.0 | 74.0 % | 60.1±18.0 | 30.8 % | 4.4 |
| Cₘₐₓ (µg/ml) | 0.8±0.3 | 37.5 % | 6.2±1.5 | 24.2% | 7.7 |
| Note : AUC = Area under the blood concentration curve Cₘₐₓ = Maximum blood concentration of cyclosporin M±S.D. = Mean value ± Standard deviation CV = Ratio of standard deviation to mean value P(B/A) = Ratio of mean value of the test preparation to mean value of the control preparation | | | | | |

As can be seen from the above table, the test preparation shows the increased AUC and Cₘₐₓ values which are about 4 times or more and about 7 times or more, respectively, as high as those of the control preparation. Accordingly, it can be identified that the bioavailability of the test preparation is clearly significantly greater than that of the control preparation. In addition, the test preparation of the present invention exhibits an effect of decreasing the difference between respective test subjects (CV %) by about 2 times or more in AUC value and by about 1.5 times in Cₘₐₓ value, in comparison with the control preparation.

Accordingly, it could be determined that when the soft capsule preparation according to the present invention is orally administered, the bioavailability of cyclosporin is about 4 times that of the prior commercial product containing ethanol, SANDIMMUN® Capsule, and also a decrease of the difference between cyclosporin bioavailabilities in respective subjects, and at the same time, remains pharmaceutically stable without any change during storage. Thus, it is apparent that the soft capsule preparation according to the present invention provides a significant improvement over prior art preparations of cyclosporin soft capsules.

## Claims

1. A cyclosporin-containing soft capsule preparation which comprises:
(1) cyclosporin as an active ingredient,
(2) propylene carbonate or polyethylene glycol or a mixture thereof as a cosurfactant,
(3) one or a mixture of two or more selected from the group consisting of an esterified compound of fatty acid and primary alcohol, medium chain fatty acid triglyceride and fatty acid monoglyceride as an oil component, and
(4) a surfactant having HLB (Hydrophilic-lipophilic balance) value of 8 to 17.

2. The cyclosporin-containing soft capsule preparation of claim 1 wherein said cyclosporin is cyclosporin A.

3. The cyclosporin-containing soft capsule preparation of claim 1 wherein said polyethylene glycol as the cosurfactant is a polyethylene glycol having molecular weight of 200 to 600.

4. The cyclosporin-containing soft capsule preparation of claim 3 wherein said polyethylene glycol as the cosurfactant is polyethylene glycol having molecular weight of 200.

5. The cyclosporin-containing soft capsule preparation of claim 1 wherein the cosurfactant is a mixture of polyethylene glycol and propylene carbonate which are combined in the ratio of 1:1-5 on the basis of weight.

6. The cyclosporin-containing soft capsule preparation of claim 5 wherein the cosurfactant is a mixture of polyethylene glycol and propylene carbonate which are combined in the ratio of 1:1-2 on the basis of weight.

7. The cyclosporin-containing soft capsule preparation of claim 1 wherein said esterified compound of fatty acid and primary alcohol as the oil component is ethyl linoleate.

8. The cyclosporin-containing soft capsule composition of claim 1 wherein said medium chain fatty acid triglyceride as the oil component is caprylic/capric acid triglyceride.

9. The cyclosporin-containing soft capsule preparation of claim 1 wherein said fatty acid monoglyceride as the oil component is a monoglyceride of oleic acid.

10. The cyclosporin-containing soft capsule preparation of claim 1 wherein the oil component is a mixture of fatty acid monoglyceride, the esterified compound of fatty acid and primary alcohol, and medium chain fatty acid triglyceride combined in the mixing ratio of 1 : 0.1-5 : 0.1-10 on the basis of weight.

11. The cyclosporin-containing soft capsule preparation of claim 1 wherein said surfactant is a polyoxyethylene product of hydrogenated vegetable oil or a polyoxyethylene-sorbitan-fatty acid ester.

12. The cyclosporin-containing soft capsule preparation of claim 11 wherein said surfactant is a mixed surfactant consisting of a polyoxyethylene(50) hydrogenated castor oil : a polyoxyethylene(20) sorbitan monolaurate in the mixing ratio of 1 : 0.1-5 on the basis of weight.

13. The cyclosporin-containing soft capsule preparation of claim 1 wherein said cyclosporin, said cosurfactant, said oil component and said surfactant are present in the ratio of 1 : 0.1-10 : 1-10 : 1-10 on the basis of weight.

14. The cyclosporin-containing soft capsule preparation of claim 13 wherein said cyclosporin, said cosurfactant, said oil component and said surfactant are present in the ratio of 1 : 0.5-8 : 2-6 : 2-8 on the basis of weight.

15. The cyclosporin-containing soft capsule preparation of claim 1 wherein the composition is encapsulated in a gelatin shell containing one or more plasticizer selected from the group consisting of glycerine, sorbitol, hexanetriol, propylene carbonate, hexane glycol, sorbitans, tetrahydrofuryl alcohol ether, diethylene glycol monoethyl ether, 1,3-dimethyl-2-imidazolidone and dimethylisosorbide.

## Patentansprüche

1. Cyclosporin enthaltende Weichkapselzubereitung, umfassend
(1) Cyclosporin als einen Wirkstoff,
(2) Propylencarbonat oder Polyethylenglycol oder ein Gemisch davon als Co-Tensid,
(3) eine Komponente oder ein Gemisch aus zwei oder mehr Komponenten, die ausgewählt sind aus der Gruppe, die besteht aus einer veresterten Verbindung aus einer Fettsäure und einem primären Alkohol, einem mittelkettigen Fettsäuretriglycerid und einem Fettsäuremonoglycerid als Ölkomponente, und
(4) einem Tensid mit einem HLB-Wert (hydrophiler-lipophiler Gleichgewichtswert) von 8 bis 17.

2. Cyclosporin enthaltende Weichkapselzubereitung nach Anspruch 1, worin das Cyclosporin Cyclosporin A ist.

3. Cyclosporin enthaltende Weichkapselzubereitung nach Anspruch 1, worin das Polyethylenglycol als das Co-Tensid ein Polyethylenglycol mit einem Molekulargewicht von 200 bis 600 ist.

4. Cyclosporin enthaltende Weicbkapselzubereitung nach Anspruch 3, worin das Polyethylenglycol als das Co-Tensid Polyethylenglycol mit einem Molekulargewicht von 200 ist.

5. Cyclosporin enthaltende Weichkapselzubereitung nach Anspruch 1, worin das Co-Tensid ein Gemisch aus Polyethylenglycol und Propylencarbonat in einem Verhältnis von 1:1-5 auf Gewichtsbasis ist.

6. Cyclosporin enthaltende Weichkapselzubereitung nach Anspruch 5, worin das Co-Tensid ein Gemisch aus Polyethylenglycol und Propylencarbonat in einem Verhältnis von 1:1-2 auf Gewichtsbasis ist.

7. Cyclosporin enthaltende Weichkapselzubereitung nach Anspruch 1, worin die veresterte Verbindung aus einer Fettsäure und einem primären Alkohol als die Ölkomponente Ethyllinoleat ist.

8. Cyclosporin enthaltende Weichkapselpräparation nach Anspruch 1, worin das mittelkettige Fettsäuretriglycerid als die Ölkomponente ein Caprylsäure-Caprinsäure-Triglycerid ist.

9. Cyclosporin enthaltende Weichkapselzubereitung nach Anspruch 1, worin das Fettsäuremonoglycerid als die Ölkomponente ein Monoglycerid von Oleinsäure ist.

10. Cyclosporin enthaltende Weichkapselzubereitung nach Anspruch 1, worin die Ölkomponente ein Gemisch aus Fettsäuremonoglycerid, der veresterten Verbindung aus Fettsäure und primärem Alkohol und dem mittelkettigen Triglycerid in einem Mischungsverhältnis von 1:0,1-5:0,1-10 auf Gewichtsbasis ist.

11. Cyclosporin enthaltende Weichkapselzubereitung nach Anspruch 1, worin das Tensid ein Polyoxyethylenprodukt aus einem hydrierten Pflanzenöl oder einem Polyoxyethylensorbitanfettsäureester ist.

12. Cyclosporin enthaltende Weichkapselzubereitung nach Anspruch 11, worin das Tensid ein gemischtes Tensid ist, das aus einem Polyoxyethylen(50)hydrierten Rhizinusöl und einem Polyoxyethylen(20)sorbitanmonolaurat in einem Mischungsverhältnis von 1:0,1-5 auf Gewichtsbasis besteht.

13. Cyclosporin enthaltende Weichkapselzubereitung nach Anspruch 1, worin das Cyclosporin, das Co-Tensid, die Ölkomponente und das Tensid in einem Verhältnis von 1:0,1-10:1-10:1-10 auf Gewichtsbasis vorhanden sind.

14. Cyclosporin enthaltende Weichkapselzubereitung nach Anspruch 13, worin das Cyclosporin, das Co-Tensid, die Ölkomponente und das Tensid in einem Verhältnis von 1:0,5-8:2-6:2-8 auf Gewichtsbasis vorhanden sind.

15. Cyclosporin enthaltende Weichkapselzubereitung nach Anspruch 1, worin die Zusammensetzung eingekapselt ist in eine Gelatinekapsel, die einen oder mehrere Weichmacher enthält, welche ausgewählt sind aus der Gruppe, die besteht aus Glycerin, Sorbit, Hexantriol, Propylencarbonat, Hexanglycol, Sorbitanen, Tetrahydrofurylalkoholether, Diethylenglycolmonoethylether, 1,3-Dimethyl-2-imidazolidon und Dimethylisosorbid.

## Revendications

1. Une préparation pour gélule molle contenant de la cyclosporine qui comprend:
(1) une cyclosporine comme substance active,
(2) du carbonate de propylène ou du polyéthylèneglycol ou un de leurs mélanges comme agent co-tensio-actif,
(3) un ou un mélange de deux ou plusieurs composés choisis parmi le groupe comprenant un composé estérifié d'un acide gras et d'un alcool primaire, un triglycéride d'acide gras à chaîne moyenne et un monoglycéride d'acide gras comme composant huileux, et
(4) un agent tensio-actif ayant une valeur du H.L.B. (balance hydrophile-liphophile) de 8 à 17.

2. La préparation pour gélule molle contenant de la cyclosporine selon la revendication 1, où ladite cyclosporine est la cyclosporine A.

3. La préparation pour gélule molle contenant de la cyclosporine selon la revendication 1, où ledit polyéthylèneglycol comme agent co-tensio-actif est un polyéthylèneglycol ayant un poids moléculaire de 200 à 600.

4. La préparation pour gélule molle contenant de la cyclosporine selon la revendication 3, où ledit polyéthylèneglycol comme agent co-tensio-actif est un polyéthylèneglycol ayant un poids moléculaire de 200.

5. La préparation pour gélule molle contenant de la cyclosporine selon la revendication 1, où l'agent co-tensio-actif est un mélange de polyéthylèneglycol et de carbonate de propylène qui sont combinés dans le rapport de 1:1-5 basé sur le poids.

6. La préparation pour gélule molle contenant de la cyclosporine selon la revendication 5, où l'agent co-tensio-actif est un mélange de polyéthylèneglycol et de carbonate de propylène qui sont combinés dans le rapport de 1:1-2 basé sur le poids.

7. La préparation pour gélule molle contenant de la cyclosporine selon la revendication 1, où ledit composé estérifié d'acide gras et d'alcool primaire comme composant huileux, est le linoléate d'éthyle.

8. La composition pour gélule molle contenant de la cyclosporine selon la revendication 1, où ledit triglycéride d'acide gras à chaîne moyenne comme composant huileux est un triglycéride acide caprylique/acide caprique.

9. La préparation pour gélule molle contenant de la cyclosporine selon la revendication 1, où ledit monoglycéride d'acide gras comme composant huileux est un monoglycéride de l'acide oléique.

10. La préparation pour gélule molle contenant de la cyclosporine selon la revendidation 1, où le composant huileux est un mélange de monoglycéride d'acide gras, de composé estérifié d'acide gras et d'alcool primaire, et de triglycéride d'acide gras à chaîne moyenne combinés dans le rapport de mélange de 1:0,1-5 : 0,1-10 basé sur le poids.

11. La préparation pour gélule molle contenant de la cyclosporine selon la revendication 1, où ledit agent tensio-actif est un produit du polyoxyéthylène d'une huile végétale hydrogénée ou d'un ester d'acide gras du polyoxyéthylène et du sorbitane.

12. La préparation pour gélule molle contenant de la cyclosporine selon la revendication 11, où ledit agent tensio-actif est un agent tensio-actif mixte constitué d'un mélange huile de ricin hydrogénée avec du polyoxyéthylène (50):monolaurate de polyoxyéthylène(20)-sorbitane dans le rapport de mélange 1:0,1-5 basé sur le poids.

13. La préparation pour gélule molle contenant de la cyclosporine selon la revendication 1, où ladite cyclosporine, ledit agent co-tensio-actif, ledit composant huileux et ledit agent tensio-actif sont présents dans le rapport de 1:0,1-10 : 1-10 : 1-10 basé sur le poids.

14. La préparation pour gélule molle contenant de la cyclosporine selon la revendication 13, où ladite cyclosporine, ledit agent co-tensio-actif, ledit composant huileux et ledit agent tensio-actif sont présents dans le rapport 1:0,5-8 : 2:6 : 2:8 basé sur le poids.

15. La préparation pour gélule molle contenant de la cyclosporine selon la revendication 1, où la composition est encapsulée dans une coque de gélatine contenant un ou plusieurs plastifiants choisis parmi le groupe comprenant la glycérine, le sorbitol, l'hexanetriol, le carbonate de propylène, l'hexaneglycol, les sorbitanes, l'éther de l'alcool tétrahydrofurylique, l'éther monoéthylique du diéthylèneglycol, le 1,3-diméthyl-2-imidazolidone et le diméthylisosorbide.
